# EUROPEAN PATENT SPECIFICATION

(11) **EP 2 501 717 B1**
(45) Date of publication and mention of the grant of the patent: **12.04.2017**
(21) Application number: 10831909.6
(22) Date of filing: 18.11.2010
(51) Int. Cl.: C07K 14/00, A61K 39/002

(54) **ENHANCED MALARIA MSP-1 SUBUNIT VACCINE**
VERSTÄRKTER MALARIA-MSP-1-UNTEREINHEITEN-IMPFSTOFF
VACCIN ANTIPALUDIQUE À BASE DE SOUS-UNITÉS DE MSP-1 AMÉLIORÉES

(30) Priority: 18.11.2009 US 281679 P
(43) Date of publication of application: 26.09.2012
(73) Proprietor: Hawaii Biotech, Inc., Aiea, HI 96701 (US); University Of Hawaii, Honolulu HI 96822 (US)
(72) Inventor: CLEMENTS, David, E., Honolulu HI 96816 (US); PUSIC, Kae, Miriam, Honolulu, HI 96815 (US); HUI, George, S., N., Honolulu HI 96825 (US)
(74) Representative: Vossius & Partner Patentanwälte Rechtsanwälte mbB
(86) International application number: PCT/US2010/003017
(87) International publication number: WO 2011/062637

(56) References cited:
- US-A1- 2002 160 017
- US-A1- 2007 003 987
- US-A1- 2008 213 318
- YUEN ET AL: "Antigenicity and immunogenicity of the N-terminal 33-kDa processing fragment of the Plasmodium falciparum merozoite surface protein 1, MSP1: Implications for vaccine development", VACCINE, vol. 25, no. 3, 8 December 2006 (2006-12-08), pages 490-499, XP005798902, ELSEVIER LTD, GB ISSN: 0264-410X, DOI: 10.1016/J.VACCINE.2006.07.053
- PUSIC KAE ET AL: "Immunological studies of Plasmodium falciparum merozoite surface protein 1, MSP1-33 and its potential influence toward MSP1 vaccine design", AMERICAN JOURNAL OF TROPICAL MEDICINE AND HYGIENE, vol. 77, no. 5, Suppl. S, November 2007 (2007-11), pages 86-87, XP009168504, & 56TH ANNUAL MEETING OF THE AMERICAN-SOCIETY-OF-TROPICAL-MEDICINE-AND- HYGIENE; PHILADELPHIA, PA, USA; NOVEMBER 04 -08, 2007 ISSN: 0002-9637
- PUSIC KAE ET AL: "DIFFERENT T EPITOPE REGIONS OF THE PLASMODIUM FALCIPARUM MSP1-33 CRITICALLY INFLUENCED THE RESPONSIVENESS, MAGNITUDE, AND QUALITY OF ANTI-MSP1-19 ANTIBODIES", AMERICAN JOURNAL OF TROPICAL MEDICINE AND HYGIENE, vol. 81, no. 5, Suppl. S, November 2009 (2009-11), page 308, XP009168505, & 58TH ANNUAL MEETING OF THE AMERICAN-SOCIETY-OF-TROPICAL-MEDICINE-AND- HYGIENE; WASHINGTON, DC, USA; NOVEMBER 18 -22, 2009
- PUSIC KAE M ET AL: "T cell epitope regions of the P. falciparum MSP1-33 critically influence immune responses and in vitro efficacy of MSP1-42 vaccines.", PLOS ONE, vol. 6, no. 9, E24782, 2011, pages 1-13, XP002694850, ISSN: 1932-6203
- NAGATA MARK ET AL: "Plasmodium falciparum: immunization with MSP1-42 induced non-inhibitory antibodies that have no blocking activities but enhanced the potency of inhibitory anti-MSP1-42 antibodies.", EXPERIMENTAL PARASITOLOGY, vol. 115, no. 4, April 2007 (2007-04), pages 403-408, XP002694851, ISSN: 0014-4894

## Description

### STATEMENT REGARDING FEDERALLY SPONSORED RESEARCH

This invention was made with government support under contract numbered 1 R43-AI51021-01 (NIH) and R21AI076955-01 (NIH). The government has certain rights in the invention.

### BACKGROUND OF THE INVENTION

### TECHNICAL FIELD

The invention relates to recombinant subunit proteins that have been designed to be used as vaccines to protect against malaria. In particular, the recombinant subunit proteins are derived from the C-terminal region of the merozoite surface protein 1 ("MSP-1") of *Plasmodium falciparum* and are further modified to enhance their immunogenic potential. The core of the MSP-1 C-terminal region is p19. The p19 core region contains epitopes that are the target of parasite growth inhibiting antibodies; however, the p19 core region by itself is poorly immunogenic. The p33 region of MSP-1 that immediately precedes the p19 core region has been demonstrated to contain T-cell epitopes. It has been hypothesized that these T-cell epitopes can enhance antibody responses directed towards the p19 core region. The functionally and the utility of these potential T-cell epitopes in the context of recombinant proteins capable of eliciting enhanced and consistent immune response has yet to be fully demonstrated. In the current application, the immunogenic potential of the p19 core region has been enhanced by the selective addition of segments derived from the p33 region of MSP-1. These selected segments have been linked to the p19 core region to produce novel proteins with enhanced immunogenic potential. These novel proteins are not found in nature. The recombinant subunit proteins with enhanced immunogenic potential are produced in a cellular production system and, after purification, are formulated as a vaccine to produce an appropriate immune response. The enhanced recombinant subunit proteins are shown to induce strong parasite growth inhibition antibodies in comparison to other MSP-1 C-terminal subunits which produce inconsistent or reduced parasite growth inhibition antibodies. The enhanced subunit proteins have the potential to be used as vaccines in humans to protect against malaria.

### RELATED ART

The annual incidence of malaria is estimated to be 250 million cases, resulting in greater than 1 million deaths annually (WHO, 2008). The majority of cases occur in Africa, although the threat extends to many other tropical and subtropical regions of the world. Approximately 3 billion people world wide are at risk of infection. At this time there is a great need to control the spread of this disease since both the mosquito vector and the *Plasmodium* parasites have developed resistance to previous control measures. Over the past ten to fifteen years, the primary focus has been on the development of malaria vaccines against the various developmental stages of the parasite. Although there has been considerable effort to establish technology for a recombinant subunit malaria vaccine and several candidate subunits have entered clinical trials, an effective malaria vaccine based on recombinant subunit protein technology has yet to be fully realized. One candidate vaccine which targets the circumsporozoite stage of malaria is still being evaluated in clinical trails, though with mixed opinions as to its potential to be advanced as a licensed vaccine. Vaccine candidates in clinical trails that target the merozoite stage of the parasite have failed thus far. As a consequence there is still no licensed vaccine available for malaria.

There are many species of malaria parasites and each species has a defined host range. There are multiple species that infect humans. *Plasmodium falciparum* is the primary species that causes disease in humans. The term "parasites" herein means *Plasmodium* species unless otherwise specified.

The life cycle of malaria parasites is complex. The parasite undergoes numerous developmental and morphological changes during the many stages of its life cycle. The cycle begins when an infected mosquito inoculates its host with sporozoites. The sporozoites quickly penetrate hepatocytes where they then develop into liver schizonts. Upon maturation, merozoites are released into the blood stream. The merozoites then invade erythrocytes where they multiply asexually until the infected cells burst, resulting in the release of additional merozoites that subsequently invade additional erythrocytes. The multiplication of the merozoites and the lysis of erythrocytes are associated with the clinical symptoms of malaria. Some merozoites go on to develop into male and female gametocytes which are then taken up by mosquitoes feeding on infected individuals. Once in the mosquito, fertilization of the female gamete by the male gamete leads to further development into the sporozoite stage of the parasite. Thus, the cycle is set to begin again. The life cycle is divided into three stages, pre-erythrocytic, asexual erythrocytic, and sexual stage. In regard to vaccine development, the three stages are often referred to as sporozoite or liver stage (pre-erythrocytic), blood stage (asexual erythrocytic), and transmission blocking (sexual stage).

While the potential exists for the development of malaria vaccines, the efforts of the past decade have proven that the development of such vaccines is a difficult task. Progress has been made in identifying and developing proteins as malaria vaccine candidates. The primary means of selecting proteins for use in malaria vaccines has been by screening for those which are reactive to human immune serum. In this way, a number of proteins in each of the life-cycle stages have been identified as vaccine candidates. The majority of these proteins that have been advanced as vaccine candidates are ones that are expressed on the surface of the parasite. Because of the difficulty in culturing parasites as a source of antigen, the development of malaria vaccines has had to rely on alternative methodologies such as recombinant DNA. This technology has been successfully utilized to define important peptide sequences, to express subunit antigens, and to develop DNA-based vaccines.

While there are efforts under way to develop malaria vaccines that target each of the developmental stages of the parasite, a vaccine targeting the blood stage is likely to have the greatest impact as this is the stage that results in disease manifestation (Good et al, 1998). Of the many blood stage antigens that have been identified, the major merozoite surface protein 1 (MSP-1) is one that has been extensively studied.

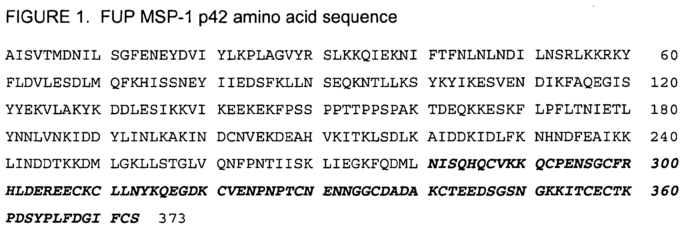

The native MSP-1 protein has a molecular weight of approximately 195 kD. It is a membrane anchored molecule that is prominently displayed on the surface of merozoites. It is processed into four major fragments, which are referred to by their relative molecular weights, p83, p28, p38 and p42 (Hall et al, 1984, Lyon et al, 1986 and Holder et al 1987). The function of all of the fragments is not known. It has been determined that the C-terminal p42 fragment, which is anchored on the surface of the merozoite, is further processed to two fragments referred to as p33 and p19, and that this processing is a prerequisite for erythrocyte invasion (Blackman et al, 1990, 1991 and Blackman and Holder, 1992). The amino acid sequence of MSP-1 p42 of the FUP strain is presented in Figure 1.

A number of findings support MSP-1 as an important malaria vaccine candidate. It has been demonstrated that monkeys can be protected from *P. falciparum* challenge when immunized with MSP-1 derived from cultured parasites (Hall et al 1984, Siddiqui et al 1987, and Etlinger et al 1991). Also, recombinant or synthetic peptides representing various portions of MSP-1 have also been shown to elicit various levels of protection in challenge studies (Hall et al, 1984, Cheung et al, 1986, Patarroyo et al, 1987, Herrera et al, 1990, Kumar et al, 1995, and Chang et al, 1996, Kumar et al, 2000, Stowers et al, 2001, Darko et al, 2005). In addition, analysis of serum collected from humans living in endemic areas has shown that the production of IgG antibodies directed at the MSP-1 C-terminal region correlates with the development of clinical immunity against falciparum malaria (Riley et al, 1992, Shai et al, 1995, Al-Yaman et al, 1996, and Shi et al, 1996). Lastly, it has been demonstrated that monoclonal antibodies against MSP-1 are capable of preventing parasite invasion of erythrocyte *in vitro* (Pirson and Perkins, 1985 and Blackman et al, 1990).

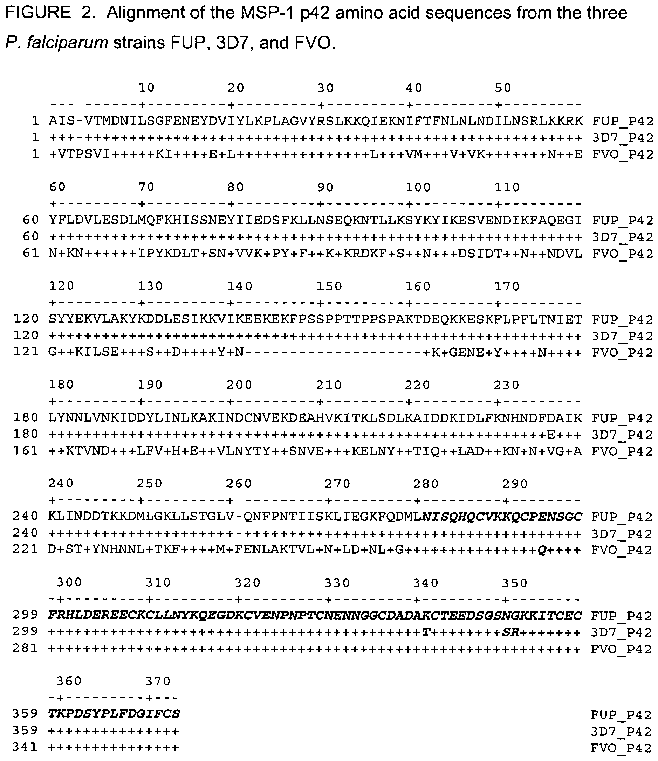

The MSP-1 C-terminal p42 fragment and its p19 processed fragment have been identified as leading subunit vaccine candidates derived from the MSP-1 protein. The sequence of the p19 core region (shown as bold and italics in Figure 1) is highly conserved among different *P. falciparum* strains. Figure 2 provides an alignment of the amino acid sequences of the MSP-1 p42 proteins from the three *P. falciparum* strains, FUP, 3D7 and FVO. The p33 region is conserved between the FUP and 3D7 strains, however, the p33 of the FVO strain is rather divergent from the other two *P. falciparum* strains. The p19 core region is highly conserved among the three strains and also contains 6 disulfide bridges that result in a highly folded structure that represents two epidermal growth factor-like domains (Blackman et al, 1991). Both polyclonal and monoclonal antibodies directed at the p19 core region have been shown to inhibit parasite *development in vitro* (Blackman et al, 1990, Chang et al, 1992, Chappel and Holder, 1993).

Efforts to develop a recombinant subunit vaccine based on the p42 or p19 fragments of MSP-1 have utilized several different heterologous protein expression systems. The expression of these subunits in *E. coli,* yeast, and baculovirus vector systems is summarized below. Early efforts to express antigens in *E. coli* induced only low levels of antibodies reactive to native protein upon immunization (Holder et al, 1988, Burghaus et al, 1996). Therefore, most efforts are currently focused on expression systems that have the capability of directing native-like folding of the MSP-1 subunits in an attempt to produce a more relevant product with greater potential to induce relevant immune responses..

Despite the ability to express more native-like MSP-1 C-terminal recombinant subunit proteins that have greater antigenic potential, all efforts to date have resulted in inconsistent and/or non-relevant antibody responses. For example, Kumar et al (1995) were able to demonstrate protection in *Aotus* monkeys when immunized with a p19 subunit that was expressed in yeast; however, protection was not achieved in all monkeys immunized demonstrating the inconsistency of the immune response with p19 subunits. Furthermore, the serum from the protected monkeys was not able to inhibit parasite growth *in vitro* indicating that the quality of the immune response (antibody specificity) was not as expected. In another set of examples, Chang et al (Infect. Immun. (1996) 64:253-261 and U.S. Patent 6,855,316) were able to demonstrate protection in *Aotus* monkeys with a p42 product produced in a baculovirus system; however, the protection was inconsistent in that not all animals were protected. This was despite the fact that the serum from protected monkeys was able to inhibit the *in vitro* growth of parasites. In yet another example, Darko et al (2005) evaluated the efficacy of a baculovirus expressed p42 product in the *Aotus* monkey challenge model. Despite relatively high level antibody responses this baculovirus expressed p42 product provided very little protection upon challenge. Lastly, a p42 product that is produced in *E. coli* and was taken through Phase II clinical trials (Ogutu et al, 2009) has now been abandoned due to low and inconsistent immune responses. From these examples, it clear that the solution for producing recombinant subunit proteins from the MSP-1 C-terminal region that are efficacious vaccine candidates has yet to be realized.

As previously described, the MSP-1 p42 fragment is composed of the p33 and p19 fragments. The p19 core region of MSP-1 which is anchored on the surface of the merozoite is a required element of the merozoite during erythrocyte invasion, where as the p33 fragment is released into the blood plasma upon binding of the merozoite to the erythrocyte. In addition to its role in erythrocyte invasion, the p19 fragment is the target of parasite growth inhibition antibodies *in vitro.* The role of the p33 fragment is not clear.

A number of T cell epitopes have been identified within the p33 region (Udhayakumar et al, 1995; Lee et al, 2001; Malhotra et al, 2008), and it has been suggested that p33 plays an indirect role in eliciting protective immunity by providing T helper epitopes that are lacking in p19 (Tian et al, 1996; Lee et al, 2001). Clinical studies have shown that memory T cell responses are elicited by MSP1-42 vaccination and these responses are localized to the MSP1-33 fragment (Huaman et al, 2008). Nevertheless, the ability of T cell epitopes to enhance an antibody response specific for p19 has not been demonstrated in the context to a recombinant subunit protein and active immunization.

In order to realize the potential of recombinant MSP-1 C-terminal subunit proteins as viable candidate vaccines, there are key issues that require further investigation. The first involves identifying the relevant immunogenic segments of the MSP-1 C-terminal region in regards to eliciting specific protective antibody responses. Specific protective antibodies are those which are capable of inhibiting parasite growth *in vitro.* The second area of investigation involves the identification of irrelevant immunogenic segments that result in responses that distract from eliciting the appropriate specific responses desired. The third area of investigation involves the evaluation of linking discontinuous sequences from the MSP-1 C-terminal region in such a manner as to produce recombinant subunit protein products that have enhanced immunogenic potential as vaccine candidates.

MSP-1 C-terminal subunit proteins are defined as any recombinant protein which contains the MSP-1 p19 core region plus additional sequences from the MSP-1 p33 region regardless of whether or not the p33 sequences are contiguous as in nature or non-contiguous as to create novel proteins.

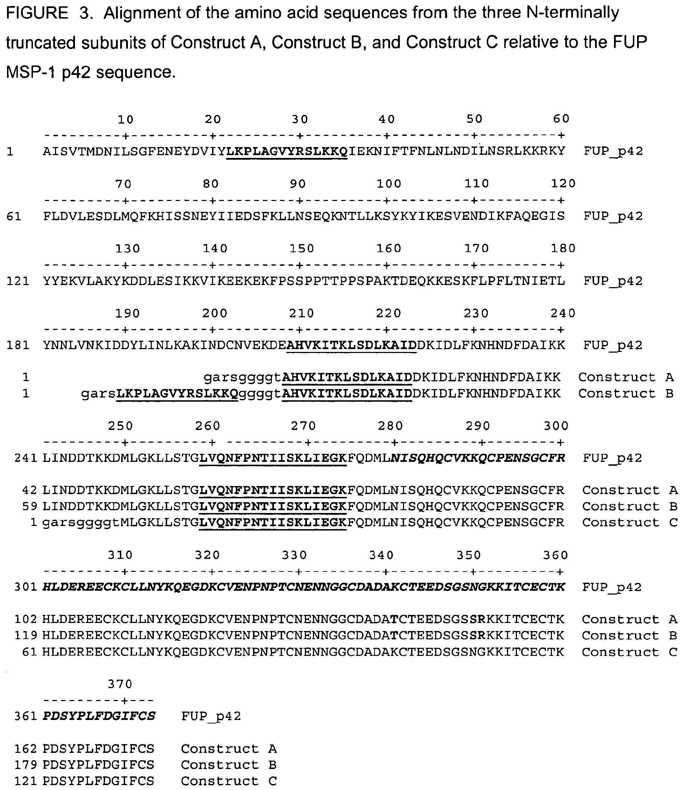

In initial efforts to develop enhanced MSP-1 C-terminal subunit proteins, three constructs were made based on N-terminal truncations of the p33 region. The truncation points were based on putative T-cell epitopes in p33. In one construct, an additional epitope from the p33 region was linked to the truncated version. The initial data from these three constructs failed to demonstrate the presence of enhancing sequences as there was no immune enhancement in terms of parasite inhibition antibodies. The sequences of these first three constructs, A, B and C are shown in Figure 3. Unexpectedly, subsequent data on Construct C suggested that the 31 amino acid p33 segment in this construct contained a sequence(s) that suppressed the immune response in terms of parasite growth inhibitory antibodies, despite a strong overall antibody response.

Therefore, further development of a viable malaria recombinant subunit vaccine based on the use of MSP-1 C-terminal region requires the identification of appropriate sequences within the p33 region that can be combined with the p19 core region in such a manner that results in the combined sequences having enhanced immunogenic properties. More specifically, the enhanced immunogenic properties refer to the ability to induce consistent and specific antibodies that are capable of parasite growth inhibition *in vitro* and providing protection against disease caused by malaria. Furthermore, in order to fully realize the potential of enhancing sequences it is also important to remove sequences that suppress the immunogenic potential of those sequences which have enhancing properties.

The selection of "enhancing" p33 sequences, in addition to the selective removal of "suppressing" p33 sequences, and the linking of the "enhancing" sequences to the p19 core region to create novel recombinant proteins capable of eliciting a consistent and enhanced protective immune response in primate animal models and humans is the ultimate goal. However, the identities of the "enhancing" and "suppressing" sequences have not previously been reported. Therefore, the technical problems to be solved are: (1) identification of sequences containing putative T cell epitopes in the p33 region that when combined with p19 core region results in an enhanced immune response ("enhancing sequences"), (2) identification and removal of sequences in the p33 regions that suppress the immunogenic potential of p19 epitopes, ("suppressing sequences") and (3) demonstrate the ability to produce recombinant subunit protein products based on the linking of discontinuous enhancing sequences from the p33 region with the p19 core region in such a manner that the products can be efficiently expressed in a cell based system. Recombinant subunit proteins produced in this manner have the potential to overcome the issues seen with previous p42 based vaccines and to consistently induce specific anti-merozoite antibodies that provide protective immunity against malaria in animal models and humans. Therefore, the overall technical problem to be solved is: the design, construction, and expression of novel recombinant subunit proteins based on sequences derived from the MSP-1 C-terminal region that result in enhanced immunogenicity and improved protective efficacy.

### SUMMARY OF THE INVENTION

The present invention relates to the embodiments characterized in the claims. Thus, it relates to the following items.
1. MSP-1 C-terminal recombinant subunit protein with enhanced immunogenic properties comprising:
   the p19 core region of the *Plasmodium falciparum* MSP-1 protein of the C-terminal region to which a segment of the p33 region is operably linked, wherein said segment of the p33 region does not include the amino acid sequence LVQNFPNTIISKLIEGK, wherein all animals immunized with said protein elicit an immunologic response that results in parasite growth inhibition activity greater than 50%, and wherein the linked segment of the p33 region is SEQ. ID. NO: 8 or SEQ. ID. NO: 14.
2. The recombinant subunit protein of item 1, wherein the protein is expressed in a eukaryotic cell culture system.
3. The recombinant subunit protein of item 2, wherein the cell culture expression system utilizes insect cells.
4. The recombinant subunit protein of item 3, wherein the insect cells are *Drosophila* S2 cells.
5. Vaccine formulations comprising MSP-1 C-terminal subunit proteins of any of items 1-4.
6. Vaccine formulations comprising MSP-1 C-terminal subunit proteins of any of items 1-4 for use in eliciting protection from malaria infection.
7. The vaccine formulations of items 5 or 6, wherein the segment of the p33 region segment of the MSP-1 C-terminal subunit protein is SEQ. ID. NO: 8 or SEQ. ID. NO: 14.

Thus, the invention provides recombinant subunit proteins that have enhanced immunogenic properties that make them suitable as vaccines to protect against malaria in animal models and humans. The recombinant subunit proteins are comprised of the MSP-1 C-terminal p19 core region of the protein and selected segments from the MSP-1 C-terminal p33 region. The addition of the selected p33 segments results in enhanced immunogenic properties. Specifically the enhanced immunogenic potential is in the ability to elicit consistent and protective antibody responses. These enhanced antibody responses have the potential to inhibit parasite growth *in vitro* and *in vivo* provide protection against disease caused by malaria parasites. The recombinant subunit proteins of the invention are expressed from transformed insect cells that contain integrated copies of the appropriate expression cassettes within the genome of the cells. The insect cell expression system provides high yields of recombinant subunit proteins with native-like conformation. Specifically, the recombinant subunit proteins are secreted from the transformed insect cells and represent novel forms of the malaria MSP-1 C-terminal region that are not found in nature. More specifically, the recombinant subunit proteins are novel derivatives that have been created to provide improved immunogenic potential to protect against disease caused by malaria parasites. In animal models, the antibodies that result from vaccination with the recombinant subunit proteins that have enhanced immunogenic potential are capable of inhibiting parasite growth *in vitro.*

The invention also provides methods for utilizing the recombinant subunit proteins described as vaccines to elicit the production of antibodies capable of conferring protection against malaria in mammalian hosts.

### BRIEF DESCRIPTION OF THE DRAWINGS

**FIG. 1****.** Amino acid sequence of MSP-1 p42 from the *P. falciparum* strain FUP (Uganda Palo Alto) of the MAD type. The p33 region is in regular type face and the p19 core region is shown in bold and italics.
**FIG. 2****.** Alignment of the MSP-1 p42 amino acid sequences from the three *P. falciparum* strains FUP, 3D7, and FVO. Amino acids in 3D7 and FVO that are the same as FUP strain are indicted with a + symbol, amino acids differing from of the FUP strain are shown, those in the p19 core region are in bold. Gaps in alignments are indicated by a - symbol. The p19 core region is shown in bold and italics
**FIG. 3****.** Alignment of the amino acid sequences from three N-terminally truncated subunits, Construct A, Construct B and Construct C relative to the FUP p42 sequence. The CT, C72, and p33-7 epitopes in the p33 sequence are indicated as bold and underlined. The p19 core region is shown in bold and italics
**FIG. 4****.** Alignment of the amino acid sequences from all MSP-1 C-terminal subunit protein constructs relative to the FUP MSP-1 p33 sequence.
**FIG. 5****.** Diagram of all MSP-1 C-terminal subunit constructs - ordered by Name
**FIG. 6****.** Diagram of all MSP-1 C-terminal subunit constructs - ordered by Group
**FIG. 7****.** ELISA antibody titers for Group 2 MSP-1 C-terminal constructs.
**FIG. 8****.** ELISPOT analysis of Group 1 and Group 2 MSP-1 C-terminal subunits

### DETAILED DESCRIPTION OF THE INVENTION

The invention provides malaria MSP-1 C-terminal recombinant subunit proteins that comprise the p19 core region and selected segments of the p33 region. These recombinant subunit proteins are produced and secreted from a stable insect cell lines that have been transformed with the appropriate expression plasmid. Immunization of animals with the recombinant subunit proteins of the invention is effective in inducing a consistent antibody response. Specifically, these antibody responses are capable of inhibiting the growth of *Plasmodium falciparum.* The combination of the selected p33 segments with the p19 core is critical for the induction of a specific and consistent immune response that results in antibodies that are capable of inhibiting parasite growth and ultimately providing protection from malaria.

In a preferred embodiment of the invention, the recombinant malaria MSP-1 C-terminal subunit proteins described herein are demonstrated to be capable of eliciting enhanced and consistent immune responses relative to MSP-1 p42 recombinant proteins which represent the form found in nature. More preferably, the enhanced recombinant MSP-1 C-terminal subunit proteins are capable of eliciting a consistent parasite inhibitory antibody response. Preferably all immunized subjects have *in vitro* growth inhibitory antibody titers greater than 50%. More preferably all immunized subjects have *in vitro* growth inhibitory antibody titers greater than 60%. Even more preferably immunized subjects have *in vitro* growth inhibitory antibody titers greater than 70%. Even more preferably immunized subjects have *in vitro* growth inhibitory antibody titers greater than 80%.

In a preferred embodiment of the invention, "enhancing" sequences are selected from the MSP-1 p33 region and linked to the p19 core region to create novel MSP-1 C-terminal recombinant subunit proteins. In addition to the selection of the "enhancing" sequences, "suppressing" sequences have also been removed from the MSP-1 p33 region to further enhance the immunogenic potential of the novel MSP-1 C-terminal recombinant subunit proteins.

In a more preferred embodiment the selected segments from the p33 region that are linked to the p19 core region of MSP-1 contain T-cell helper epitopes. These T-cell helper epitopes are responsible for directing the enhanced immune response toward the p19 core region which in of itself is poorly immunogenic. More preferably the selected p33 segments do not contain sequences and or epitopes that inhibit or suppress the ability to elicit enhanced antibody responses in the form of inhibitory antibodies directed against the p19 core region. Therefore, the resultant recombinant proteins differ from the native p33 region in that only the segment(s) that provide benefit are retained and those that have a negative impact have been removed.

In another preferred embodiment of the invention, the recombinant malaria MSP-1 C-terminal subunit proteins comprise the p19 core of MSP-1 and selected p33 segments which are linked in such a manner that the recombinant proteins can be expressed as a secreted product in cell based expression system. The secreted recombinant subunit proteins are then purified and used to as immunogens. The purified proteins result in enhanced immune responses when used to immunize animals.

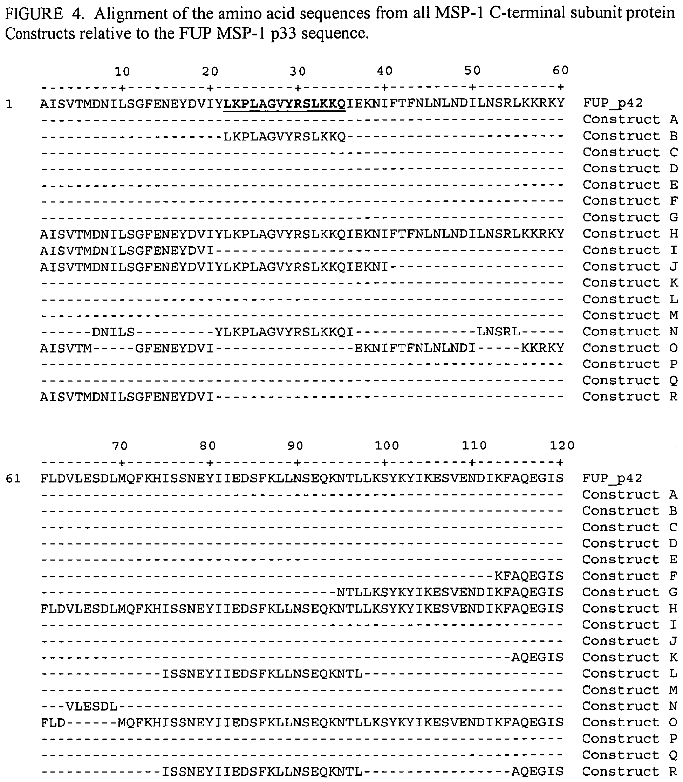

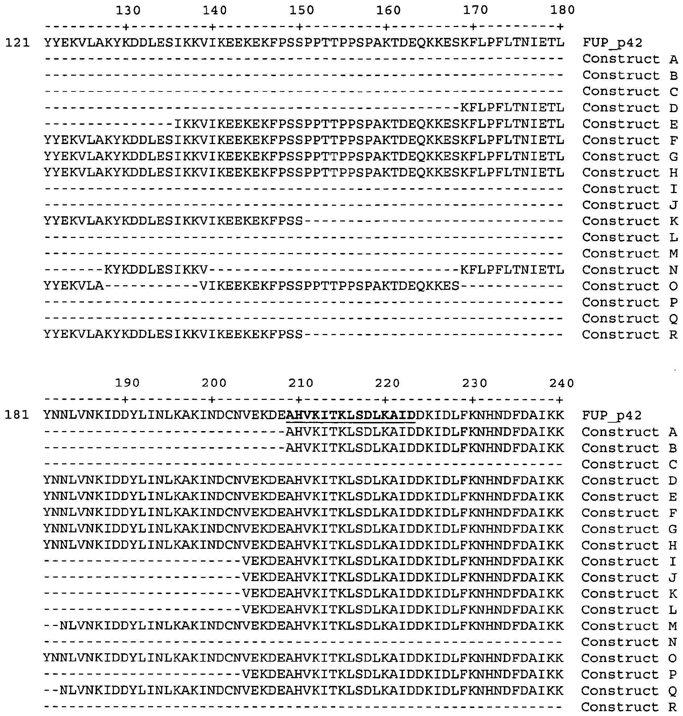

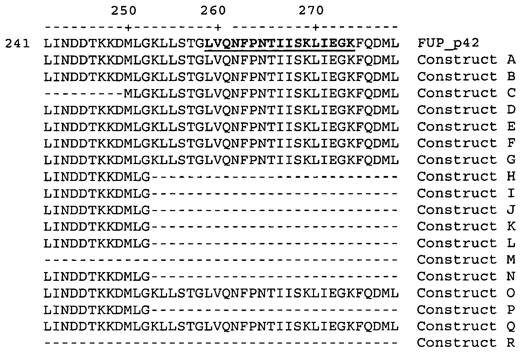

In yet another preferred embodiment "MSP-1 C-terminal subunits proteins" are expressed using *Drosophila* S2 cells as host cells. "MSP-1 C-terminal subunits proteins" refer to As disclosed herein, "MSP-1 C-terminal subunits proteins" refer to any recombinant protein which contains the MSP-1 p19 core region plus additional sequences from the MSP-1 p33 region regardless of whether or not the p33 sequences are contiguous as in nature or non-contiguous as to create novel proteins. These "MSP-1 C-terminal subunit proteins" can be derived from any strain of *Plasmodium falciparum.* Figure 4 discloses the segments of p33 sequences utilized in all of the "MSP-1 C-terminal subunit proteins" referred to in this application.

As disclosed herein, subunit proteins comprising MSP-1 C-terminal p19 core region and segments derived form the MSP-1 C-terminal p33 region were expressed and secreted from stably transformed *Drosophila* S2 cells by operably linking the coding sequences of such proteins to functional secretion signals and placing them under the control of a promoter demonstrated to be functional in *Drosophila* cells utilizing standard recombinant DNA methods.

The recombinant MSP-1 C-terminal subunit proteins described herein represent three different strains derived from of the species *Plasmodium falciparum.* The sequences encoding the MSP-1 C-terminal subunit proteins from the two strains of *P. falciparum,* i.e., FUP (Uganda Palo Alto) of the MAD type, NF-54 (clone 3D7) of the Wellcome type and the FVO (Vietnam-Oak Noll) of the K1 type. All sequences were cloned into *Drosophila* expression plasmids and then used to transform *Drosophila* S2 cells.

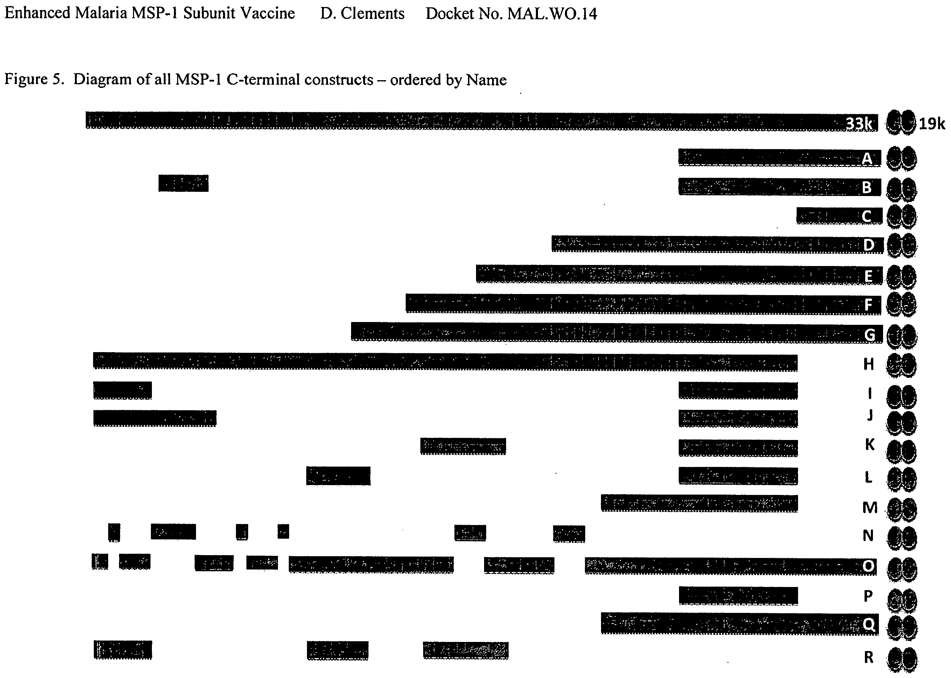

Thus, the MSP-1 C-terminal subunits as disclosed herein are all related as they all contain the MSP-1 C-terminal p19 core region as depicted in Figure 5. Furthermore, while the p33 sequences vary in each construct, all constructs are designed to direct and enhance the potency and the specificity of the immune response to the p19 core region. The enhanced quality of the antibody response to the p19 core region is associated with an increased ability to provide protection against disease that results from malaria infection.

The MSP-1 C-terminal subunit proteins as disclosed herein are expressed and secreted from selected S2 cell lines as described are first purified by immunoaffinity chromatography methods. The anti-MSP-1 monoclonal antibody 5.2 (Chang et al, 1992) ("5.2 antibody" or "MAb 5.2") is utilized for the purification. The 5.2 antibody is chemically conjugated to the appropriate column matrix by standard methods recommended by the manufacturer (NHS-Sepharose, Pharmacia, Piscataway, NJ) to prepare suitable columns.

As disclosed herein, the MSP-1 C-terminal subunits are derived from the portion of the P. *falciparum* merozoite surface protein referred to as p42; in the FUP and 3D7 strains, p42 comprises amino acids Ala₁₃₃₃ to Ser₁₇₀₅, of MSP-1. The MSP-1 C-terminal subunit proteins, as described herein, are recombinantly produced and secreted from stably transformed insect cells. The MSP-1 C-terminal subunits may contain the entire p42 region of MSP-1 or portions thereof. As disclosed herein, MSP-1 C-terminal subunits contain the MSP-1 p10 core region linked to MSP-1 p33 sequences that are either contiguous or non-contiguous relative to the native p42, but all MSP-1, but all MSP-1 C-terminal subunit proteins area t least 28 amino acids shorter in length than the native p42 of the FUP and 3D7 strains. The MSP-1 C-terminal subunits can be are derived from any of the three allelic types of *P. falciparum;* such as: K, MAD20, and Wellcome, as well as allelic types of *P. vivax, P. malariae* and *P. ovale.*

As disclosed herein, the secretion of the MSP-1 C-terminal subunit proteins is directed by a functional secretion signal capable of directing the expressed product through the insect cell secretion pathway and into the culture medium. Here in, the secretion of MSP-1 C-terminal subunit proteins is demonstrated utilizing the tPA pre/pro secretion leader and a synthetic secretion signal.

Surprisingly, and in contrast to previously described reports of MSP-1 p19 and MSP-1 p42 recombinant subunits where it is thought that very high antibody titers are a key indicator of parasite growth activity, the ability of several of the recombinant MSP-1 C-terminal subunits described in this application to elicit significant parasite growth inhibition activity is not dependent on the need to generate high titer antibody responses. It was found for several of the MSP-1 C-terminal subunit proteins that elicited moderate antibody titer also resulted in significant parasite growth inhibition activity. Thus, the MSP-1 C-terminal proteins described are novel in that they are functionally different relative to MSP-1 based recombinant proteins previously described in theie ability to elicit significant parasite growth inhibition activity.

The present invention thus concerns and provides recombinant subunit proteins that can be used in vaccine formulations as a means for preventing or attenuating infection by *Plasmodium* species. The recombinant subunit proteins described may be used in combination with adjuvants as a means to provide protection against disease caused by malaria infection.

Although the descriptions presented above and the examples that follow are primarily directed at the expression of MSP-1 C-terminal subunits from *Plasmodium falciparum,* the methods can be applied to other *Plasmodium* species. For example *P. vivax, P. malariae* and *P*. *ovale* species that also pose a health threat to humans. Constructs analogous to those for *P*. *falciparum* described herein can be developed for *P. vivax, P. malariae* and *P. ovale,* respectively, for use as vaccines to prevent disease caused by malaria infection.

### EXAMPLES

The examples below demonstrate the ability to select and remove sequences from the MSP-1 p33 region and link the selected sequences to the MSP-1 p19 core region so as to produce novel recombinant subunit proteins that have enhanced immunogenic potential as vaccine candidates. The expression and secretion of the novel MSP-1 C-terminal subunit proteins is also demonstrated utilizing stably transformed insect cells. The purification of the expressed subunit proteins is also demonstrated. Examples are provided that demonstrate the enhanced immunogenic properties of the novel MSP-1 C-terminal subunit proteins. The results presented below demonstrate that the selection and demonstration of enhanced immunogenic potential results in highly variable results that can vary from a loss of a specific immune response to highly focused immune responses. Thus, the selection of enhancing sequences from the p33 region in an effort to focus the immune response the p19 core region is not entirely predictable and must be determined empirically. Several of the MSP-1 C-terminal subunit proteins unexpectantly were able to elicit enhanced significant immunogenic properties, parasite inhibition activity greater than 50%, despite the fact that the novel recombinant proteins produced moderate general antibody responses. Hence, the invention described herein is unique in the composition of the recombinant subunit proteins described and in their resultant immunogenic properties.

The following examples are intended to illustrate but not to limit the invention.

### EXAMPLE 1

### Assessment of T-cell epitopes in the p33 region of MSP-1 p42

There are many studies that have utilized either the p19 or p42 subunits of MSP-1 in an effort to develop a malaria vaccine. There have been reports of success for each of these MSP-1 subunits as well as failures with each of these subunits, as reviewed above and further discussed below. Because of the conflicting results, it is not clear which regions of these subunits are most relevant in regards to their ability to serve as antigens in eliciting antibody responses that are capable of inhibiting parasite growth *in vitro* or in the protection of animals to challenge. It has been hypothesized that the induction of growth-inhibitory antibodies to the p19 core region is determined by the specificity of T helper epitopes in p33 region. In support of this theory, a study that directly compared the ability of recombinant p19 and p42 subunits to elicit parasite growth inhibitory antibodies was conducted (Hui et al, 1994). Using the same adjuvant (FCA), both p19 and p42 recombinants elicited high titers of antibodies based on ELISA determination. However, the antibodies generated by the p42 antigen were inhibitory against parasite growth were as the antibodies generated by the p19 were incapable of inhibiting parasite growth. Despite the inability of the p19 generated antibodies to inhibit parasite growth, it was demonstrate that of the p42 generated antibodies capable of binding to parasite-derived MSP-1 could be cross-absorbed using the p19 antigen. Also in support of this theory, a study by Udhayakumar et al (1995) characterized B and T cell epitopes in the p42 region relative to the immune response of individuals living in Kenya, a region in which falciparum malaria is highly endemic. This study revealed that B cell epitopes were mainly in the p19 core region and that T cell epitopes capable of providing proliferative responses were located in the p33 region. Studies by Lee et al (2001) and Malhotra et al (2008) have also identified other potential T-cell epitopes in the p33 region that may be involved in enhancing protective immune responses for the MSP-1 C-terminal region.

While the T cell epitopes identified in these studies were not determined to directly provide T-cell helper function, it is suggested that they may be responsible for focusing the antibody response to the important parasite growth inhibiting epitopes of p19. While these results support the theory of T-cell helper functions in the p33 region, there is no data on the expression of recombinant subunits that are designed to enhance the ability to elicit parasite growth inhibitory antibodies or protective responses in animal models.

Computer programs were used to aid in the analysis to guide the selection of appropriate segments of p33 to retain relevant T-cell epitopes. First, the p33 region was analyzed for the presence of sequences that fit the pattern established for T-cell epitopes (Margalit et al, 1987). The algorithm is part of a computer program written by Menendez-Arias and Rodriguez (1990) for selecting potential T-cell epitopes. This analysis resulted in the prediction of 14 T-cell epitopes. The epitope with the highest amphipathic score is one that is in the conserved region at the N-terminus of p33, LKPLAGVYRSLKKQ. This epitope is referred to as CT (conserved T). The next epitope that was identified is positioned 72 amino acids preceding the start of the p19 sequence, AHVKITKLSDLKAID, and is referred to as C72. A third T-cell epitope was identified based on further analysis of MHC class II epitopes in the p33 region with the computer program TEPITOPE (Sturniolo T. et al, Nat. Biotechnology (1999) 17:555-561; Singh,H. and Raghava,G.P.S.(2001) Bioinformatics, 17(12), 1236-37). This epitope is positioned 22 amino acids preceding the start of the p19 sequence, LVQNFPNTIISKLIEGK, and is referred to as p33-7

Table 1 below list potential MHC class II T-cell epitopes in the p33 region of MSP-1 p42 of *P. falciparum.* These epitopes are predicted by computer algorithms or are based on published reports as described above.

**Table 1. Potential T-cell epitopes in the p33 region of MSP-1 p42**

| **Peptide** | **Amino Acid Sequence** | **Position of peptide in p42** | **Peptide Length** |
|---|---|---|---|
| CT | LKPLAGVYRSLKKQIEK | 23-38 | 17 |
| CT-2 | VIYLKPLAGVYRSLKKQIE | 19-37 | 19 |
| p33-2 | FNLNLNDILNSRLK | 43-56 | 14 |
| p33-3 | LMQFKHISSNEYIIEDS | 69-85 | 17 |
| p33-4 | FLPFLTNIETLYNNLVNKID | 170-189 | 20 |
| p33-5 | LYNNLVNKIDDYLINLKAKIND | 180-201 | 22 |
| p33-6 | YLINLKAKIND | 191-201 | 11 |
| C72 | AHVKITKLSDLKAID | 209-223 | 15 |
| p33-7 | LVQNFPNTIISKLIEGK | 259-275 | 17 |
| p19-1 | FQDMLNISQHQCVKK | 276-292 | 15 |

### EXAMPLE 2

### Expression and Secretion of MSP-1 C-terminal Subunit Proteins - p33 N-terminal truncations

The first group of MSP-1 C-terminal subunit proteins is based on N-terminal truncations of the p33 region of the MSP-1 p42 protein in an effort to define regions of p33 that could enhance the ability to elicit parasite growth inhibitory antibodies in animal models. The truncation points were guided by the location of the various potential T-cell epitopes present in Table 1. As these constructs were based on N-terminal deletions, the portions of p33 retained were maintained together with the p19 region in contiguous manner as in the native p42 as exemplified in Figure 3. A total of eight constructs were made based on the N-terminal truncations, Constructs A, B, C, D, E, F, G, and Q.

The design of the expression plasmids for the expression and selection of heterologous recombinant proteins in cultured *Drosophila* S2 cells is described. The expression of MSP-1 p42 and Constructs A, B and C was accomplished utilizing pMttbns based expression plasmids. The details related to these expression plasmids are provided in U.S. Patent Numbers: 5,550,043; 5,681,713; 5,705,359; 6,046,025. The pMttbns expression vector contains the following elements: the *Drosophila* metallothionein promoter (Mtn), the human tissue plasminogen activator (tPA) signal sequence, and the SV40 early polyadenylation signal (Culp et al, 1991). The pCoHygro plasmid is also utilized as selectable marker that confers hygromycin resistance (Van de Straten, 1989). The hygromycin gene is under the transcriptional control of the *Drosophila* COPIA transposable element long terminal repeat promoter. The pMttbns vector was modified by deleting a 15 base pair BamHI fragment which contained an extraneous Xho I site. This modified vector, referred to as pMttΔXho, allows for directional cloning of inserts utilizing unique Bgl II and Xho I sites.

The expression of Constructs D, E, F, G, and Q was accomplished using the *Drosophila* expression plasmid pHBI-20D which is a derivative of the plasmid pHBI-10. The details of the pHBI-10 plasmid are provided in PCT Application WO/2008/134068. The pHBI-20D expression vector contains the following elements: synthetic metal responsive proximal promoter, synthetic core promoter and 5'UTR, synthetic secretion signal, and an optimized 3'UTR. The hygromycin encoding gene is also incorporated into the pHBI-20D expression plasmid downstream of the expression cassette. The pHBI-20D expression plasmid is designed to allow directional cloning of the gene of interest into unique Bam HI and Xho I sites.

For the expression of MSP-1 p42 sequences, PCR amplified fragments were prepared and cloned into the expression vector pMttΔXho. Genomic DNA was prepared from cultured *P*. *falciparum* parasites of the three strains, FUP, NF54 (clone 3D7) and FVO utilizing the DNeasy Tissue Kit from Qiagen (Valencia, CA).

The full MSP-1 p42 and the MSP-1 C-terminal fragments for Constructs A, B, C, D, E, F, G and Q were generated by PCR amplification with oligonucleotide primers. The amplified MSP-1 p42 PCR fragment contains the sequence encoding amino acids Ala₁₃₃₃ to Ser₁₇₀₅ of MSP-1 for the FUP (Genbank accession number M37213). All of the N-terminal truncations were amplified from the full length MSP-1 p42 construct. The oligonucleotide primers used for amplification also encoded for appropriate restriction sites and stop codons. Construct B varies for the other N-terminally truncated subunits which all have contiguous sequences derived from p42 in that the CT epitope described in example 1 is fused to the N-terminus of Construct A.

PCR amplification was accomplished by use of the high fidelity Pfx polymerase (Invitrogen, Carlsbad, CA). The resultant PCR amplified fragments were digested with appropriate restriction enzymes and inserted into the pMttΔXho vector digested with Bgl II and Xho I or into the pHBI-20D vector digested with Bam HI and Xho I. The junctions and full inserts of all constructs were sequenced to verify that the various components that have been introduced are correct and that the proper reading frame has been maintained.

*Drosophila melanogaster* S2 cells ("*Drosophila* S2 cells" or simply "S2 cells" Schneider, 1972) obtained from ATCC were utilized. Cells are adapted to growth in Excell 420 medium and all procedures and culturing are in this medium. Cells are passed between days 5 and 7 and are typically seeded at a density of 1x10⁶ cells/mL and incubated at 26°C. All expression plasmids containing the coding sequences for the MSP-1 C-terminal subunit proteins were transformed into S2 cells by means of the calcium phosphate method. When the pMttΔXho plasmid was used the cells were co-transformed with the pCoHygro plasmids for selection with hygromycin B at a ratio of 10 µg of expression plasmid to 0.5 µg of pCoHygro. When the pHBI-20D plasmids were used the cells were transformed with only 10 µg the expression plasmid as the hygromycin gene is incorporated into this plasmid. Following transformation, cells resistant to hygromycin, 0.3 mg/mL, were selected. Once stable cells lines were selected, they were evaluated for expression of the appropriate products. Five mL cultures were seeded at 2x10⁶ cells/mL and induced with 0.2 mM CuSO₄ and cultured at 26°C for 7 days. After the removal of cells, samples of culture medium were subjected to SDS-PAGE and Western blot analysis to evaluate expression of the various MSP-1 C-terminal subunit proteins.

Immunoaffinity chromatography (IAC) methods were utilized as a rapid means to purify expressed MSP-1 C-terminal subunit proteins for preliminary antigenic and immunogenic studies. The MSP-1 conformationally sensitive mAb 5.2 was used to prepare p42-specific IAC columns (Siddiqui et al 1987 and Chang et al 1992). Sufficient quantities of the mAb 5.2 were produced in a Cell Pharm 1000 hollow fiber bioreactor according to the manufacturer's recommendations (Unisyn, Hopkinton, MA). The mAb 5.2 hybridoma cell line was obtained from ATCC and grown in RPMI 1640 (Cambrex, Hopkins, MA). The medium was supplemented with FBS at 10% for growth in flask and 5% for growth in the hollow fiber bioreactor. The bioreactor was run for 25 days and resulted in a total yield of 57 mg. A two mL bed volume column was made by coupling 10 mg of affinity purified MAb 5.2 per mL of column matrix (activated N-hydroxy-succinimide-HiTrap, Pharmacia, Piscataway, NJ). The IAC column was perfused with 200 mL of clarified culture medium at a rate of 1 mL per minute. Following washing with 10 mM phosphate buffer, pH 7.2, the antigen was eluted with 100 mM glycine pH 2.5. The eluted product was neutralized with 1 M Tris, pH 7.5 (final concentration 0.2 M), and NaCl was added to a final concentration of 150 mM. The sample was then buffer exchanged into phosphate buffered saline ("PBS") and concentrated by membrane ultrafiltration using a Centricon 30 (Millipore, Bedford, MA).

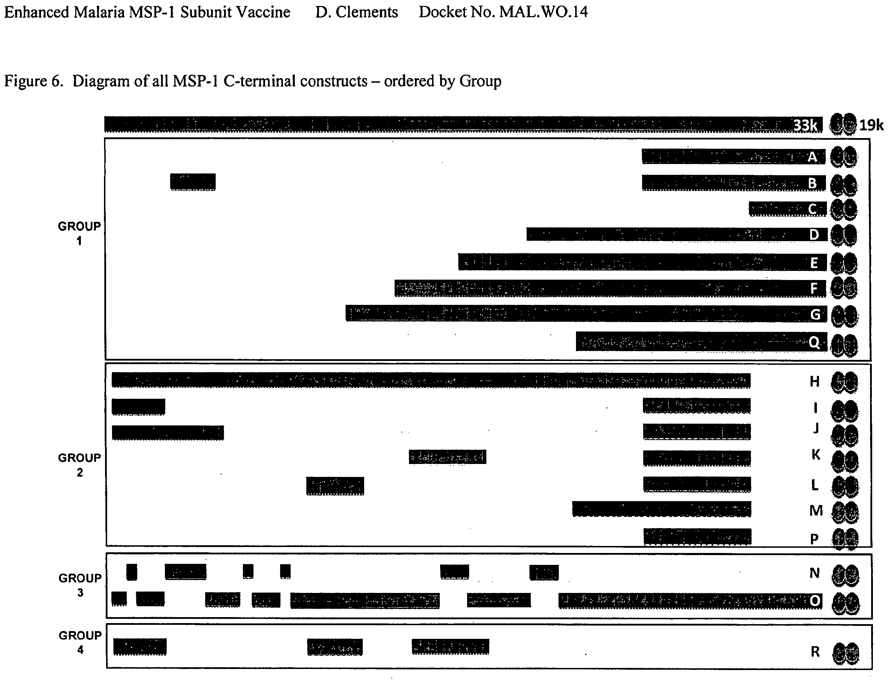

The N-terminal constructs are shown in Figures 4, 5 and 6. As seen in Figure 6, the N-terminal truncation constructs are referred to as Group 1. Group 1 constructs are defined as any construct which retains a single continuous portion of the C-terminus of p33 immediately preceding p19 as in the native p42 sequence. The Group 1 constructs with the N-terminal truncations represent MSP-1 C-terminal subunits that are novel in that these subunits are not found in nature due to cellular processing.

### EXAMPLE 3

### Evaluation of the Immunogenicity of MSP-1 C-terminal Subunit Proteins Constructs A, B and C

The immunogenicity of the *Drosophila* expressed MSP-1 C-terminal subunit proteins which were based on p33 N-terminal truncations (Group 1) were evaluated in mice and rabbits to determine if they had enhanced immunogenic potential. For all initial experiments, either Freund's complete Adjuvant (FCA) or ISA51 was utilized to deliver the subunit proteins.

Mice were immunized intra-peritoneally three times at a 4 week interval with the purified subunit proteins at a 10 µg dose. Mice were bled three weeks after each dose. The sera were then assessed for anti-p19 and anti-p42 titers by ELISA. New Zealand White rabbits were immunized four times at a 4 week interval by the intramuscular route with a 50 µg dose. Rabbits were bled two weeks after the first three doses and at three and four weeks after the fourth dose. The sera were then assessed for anti-p42 titers by ELISA. The sera from rabbits were also tested in an *in vitro* assay for parasite growth acitivity (Hui et al., Exp.Parasitol, (1987) 64:519-522). Briefly, to assay for parasite growth inhibition activity, the serum sample is added to culture medium to give a final concentration of 30% and incubated with infected human erythrocytes that are adjusted to an initial parasitemia of 0.5%. *P. falciparum* 3D7 parasites that are adapted to growth in human serum are utilized in the assay. Cultures are then incubated for 72 hours, and the parasitemia of Giemsa-stained thin smears of the cultured erythrocytes are determined by microscopy. The percent inhibition is calculated by subtracting the parasitemia in test samples from the parasitemia in control serum samples (pre-bleeds from the same animal) and dividing by the parasitemia in control serum sample and then multiplying by 100.

An example of the results obtained when MSP-1 p42 formulated with Freund's Complete Adjuvant (FCA) is used to immunize rabbits is presented in Table 2. The results from the MSP-1 p42 immunized rabbits demonstrate that the p42 vaccine formulation has the potential to elicit appropriate immune response, high ELISA titers and parasite growth inhibition titers greater than 50%. These results can only be obtained with strong adjuvants such as FCA. However, FCA is not suitable for use in humans and so these results do not describe a formulation that is a potential vaccine for humans. Use of other adjuvants that have potential for use in humans results in inconsistent parasite growth inhibition antibodies, despite good overall antibody titers upon immunization. Consequently it is necessary to develop alternative means to enhance the immunogenic potential of recombinant subunit proteins that can direct consistently strong and specific immune responses, parasites growth inhibitory antibodies.

**Table 2. ELISA titration and inhibition results on serum from rabbits immunized with MSP-1 p42 formulated with Freund's Complete Adjuvant**

| Rabbit ID | MSP-1 p42 | % inhibition |
|---|---|---|
| 7866 | 3,125,000 | 84 |
| 7867 | 625,000 | 60 |
| 7868 | 3,125,000 | 87 |

| | | |
|---|---|---|
| * dilution (k = x 1000) which gives an O.D. (Optical Density) of 0.1 above background (O.D. values reported are after subtraction of background) | | |

In Table 3 ELISA data is presented for Constructs A , B and C. Based on the ELISA titers which only measure the overall antibody response rather than the specific antibody response, Construct C results in the strongest and consistent immune response.

**Table 3. ELISA titers of serum from rabbits immunized with recombinant MSP-1 C-terminal constructs A, B and C.**

| **Construct** | **Rabbit #** | **ELISA Titers** | |
|---|---|---|---|
| | | **MSP-1 p42** | **MSP-1 p19** |
| A | Rbt#1 | 624,000 | 93,000 |
| | Rbt#2 | 361,000 | 23,000 |
| B | Rbt#1 | 27,000 | 161,000 |
| | Rbt#2 | 156,000 | 137,000 |
| | Rbt#3 | 254,000 | 121,000 |
| C | Rbt#1 | 2,490,000 | 254,000 |
| | Rbt#2 | 948,000 | 385,000 |
| | Rbt#3 | 1,265,000 | 498,000 |

In Table 4 parasite growth inhibition data is presented for Constructs A , B and C. Based on the inhibition data which measures the specific antibody response, Construct A and B were able to produce a specific response in immunized animals, though in an inconsistent manner as often seen for p19 and p42 subunits. On the other hand, Construct C failed to generate any appreciable inhibition antibodies (only results greater than 50% are considered to be significant based on the variability of the assay). The results from Construct C were totally unexpected. Prior to this result a key working premise was that consistent and high overall antibody titers were a prerequisite for the generation of specific inhibitory antibodies. Furthermore, the results from Construct C strongly suggested that there are p33 sequences that can suppress the generation of anti-p19 antibodies that are capable of parasite growth inhibition. This is despite the fact that the p33 segment in Construct C has a predicted T-cell epitope (p33-7 in Table 1 and as shown in Figures 3 and 4) as predicted by the :PROPED (TEPITOPE) algorithm (Singh et al., Bioinformatics, (2001) 17:1236-7; Sturniolo et al., Nat Biotechnol. (1999) 17:555-61).

**Table 4. Percent inhibition of parasite growth with serum from rabbits immunized with recombinant MSP-1 C-terminal constructs A, B and C.**

| **Construct** | **Rabbit #** | **% Parasite Growth Inhibition** |
|---|---|---|
| Construct A | Rbt#1 | 75% |
| | Rbt#2 | 37% |
| Construct B | Rbt#1 | 66% |
| | Rbt#2 | 23% |
| | Rbt#3 | 0% |
| Construct C | Rbt#1 | 10% |
| | Rbt#2 | 32% |
| | Rbt#3 | 22% |

The parasite inhibition results from Constructs A, B and C demonstrate that MSP-1 C-terminal constructs made based on the prediction of potential T-cell epitopes alone is not enough to ensure that enhanced immune responses specific for the p19 region will be generated. In fact, the unexpected result with Construct C demonstrated that at least one of the predicted epitopes, p33-7, can greatly suppress the specific antibody response desired. Thus, predicting the appropriate nature of the constructs that have enhancing immune potential requires considerable experimentation to sort out which sequences may be enhancing and which may be suppressing.

### EXAMPLE 4

### Expression and Secretion of MSP-1 C-terminal Subunit Proteins - p33 linked sequences

Additional MSP-1 C-terminal subunit proteins were made based on combinations of discontinuous segments from the p33 region of the MSP-1 p42 protein in an effort to link together enhancing sequences while excluding suppressing or extraneous sequences in regards to the ability to elicit parasite growth inhibitory antibodies in animal models. As with Group 1 constructs the selection of p33 segments was in part guided by the location of the various potential T-cell epitopes presented in Table 1. Three different groups of constructs containing non-continuous segments were made, Group 2, Group 3 and Group 4. In addition to selecting fragments with known T cell epitopes there were two additional design criteria for Group 2 constructs. The first was the exclusion the p33 segment in Construct C, based on the data in Example 2 that this segment as it contains suppressing sequences. The second was only two to three fragments were linked. As the Group 2 constructs are based on discontinuous segments of p33, the resultant constructs in which the p33 sequences are linked to p19 are novel in that the combined sequences are not found in nature. A total of seven Group 2 constructs were made. Six constructs were based on the linkage of various combinations of p33 segments with the p19 region, and the exclusion of the Construct C region, Constructs I, J, K, L, M, and P. One construct, H, was based only on the exclusion of the Construct C region. Group 3 constructs were based on linking either all of the conserved fragments of the p33 region, Construct N, or linking all of the non-conserved fragments, Construct O. Both of these constructs were made by synthetic methods. Group 4 contains only one Construct, R. Based on preliminary data, selected fragmentst from constructs I, K, and L where selected and combined.

The pHBI-20D *Drosophila* expression plasmid described in Example 1 was used for all Groups 2, 3 and 4 constructs. In order to make the Group 2 and 4 constructs a pHBI-20D-p19 base plasmid was first constructed. The pHBI-20D-p19 base plasmid contains the full p19 sequence which is immediately preceded by sequence that encodes Gly-Gly-Thr-Gly-Gly-Gly linker. The sequence contains a unique Kpn I restriction site that was utilized to link the p33 sequences in frame with the p19 sequence. The Bam HI site which is the typical cloning site at the end of the secretion signal sequence in pHBI-20D was also modified to a Nhe I site to allow for multiple cloning events of the various p33 sequences that were combined. Expression constructs were made by directly cloning PCR amplified fragments, or subcloning fragments from shuttle vectors containing fragments that were chemically synthesized, into the expression vector pHBI-20D-p19. For Group 3 constructs which were made synthetically due to their complexity of non-continuous segments of p33, the p19 region was also include in the synthesis. Therefore, there are no linker sequences for Group 4 constructs between the p33 fragments or the p33 and p19 region. The synthetic fragments were cloned directly into pHBI-20D with appropriate restriction enzymes.

Oligonucleotide primers and synthetic sequences were designed based on the FUP sequence (Genbank accession number M37213). In addition to the MSP-1 specific sequences, the oligonucleotide primers encoded for appropriate restriction sites. For the linked segments the 5' end of the fragments were designed with a Spe I site (compatible with Nhe I) and the 3' end of the fragments were designed with a Kpn I site to correspond with the cloning sites in pHBI-20-p19. When two p33 fragments were combined they were linked by designing Bam HI sites at the appropriate ends to allow for linkage. The PCR amplification, cloning, and transformation of S2 cells were accomplished as described in Example 2. The expressed subunit proteins were purified by IAC methods as described in Example 2.

The p33 linked constructs representing Groups 2, 3 and 4 are shown in Figures 4, 5 and 6. Group 2 constructs are defined as any construct which has one ore more discontinuous segments of p33 linked to the p19 region. The resultant constructs in which the p33 sequences are linked to p19 are novel in that the combined sequences are not found in nature.

### EXAMPLE 5

### Evaluation of the Immunogenicity of MSP-1 C-terminal Subunit Proteins in Mice

The immunogenicity of the *Drosophila* expressed MSP-1 C-terminal subunit proteins which were based on p33 linked sequences (Group 2) were evaluated in mice and rabbits to determine if they had enhanced immunogenic potential. For all initial experiments, either Freund's complete Adjuvant (FCA) or ISA51 was utilized to deliver the subunit proteins.

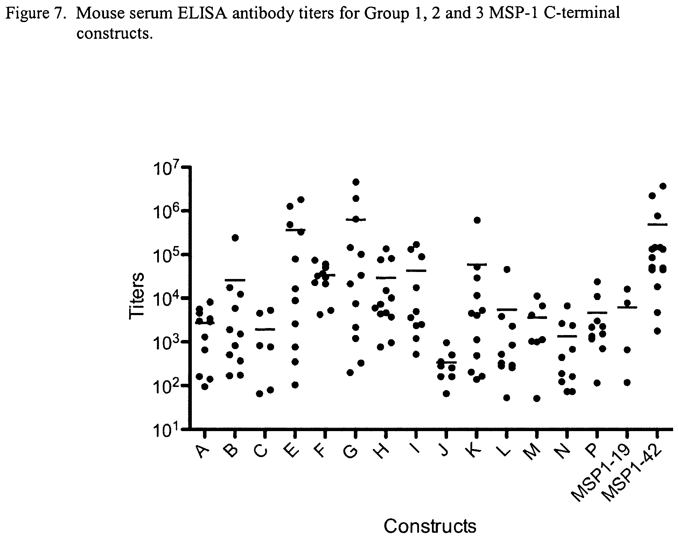

The immunization of mice and rabbits and the evaluation of the immune responses were conducted as described in Example 3. The antibody titers for the Group 2 constructs are presented in Figure 7. These results indicate that there are various levels of antibody titers induced by these constructs with constructs H and I having the highest titer and construct J having the lowest titers.

Table X. Percent responders for groups of mice immunized with Group 1, 2 and 3 MSP-1 C-terminal subunit proteins based on reactivity in a p19 specific ELISA.

| **Construct** | A | B | C | D | E | F | H | I | J | K | L | M | N | P | p42 | p19 |
|---|---|---|---|---|---|---|---|---|---|---|---|---|---|---|---|---|
| **% Responders** | 54 | 62 | 22 | 82 | 80 | 100 | 100 | 80 | 62 | 100 | 82 | 42 | 80 | 80 | 100 | 18 |

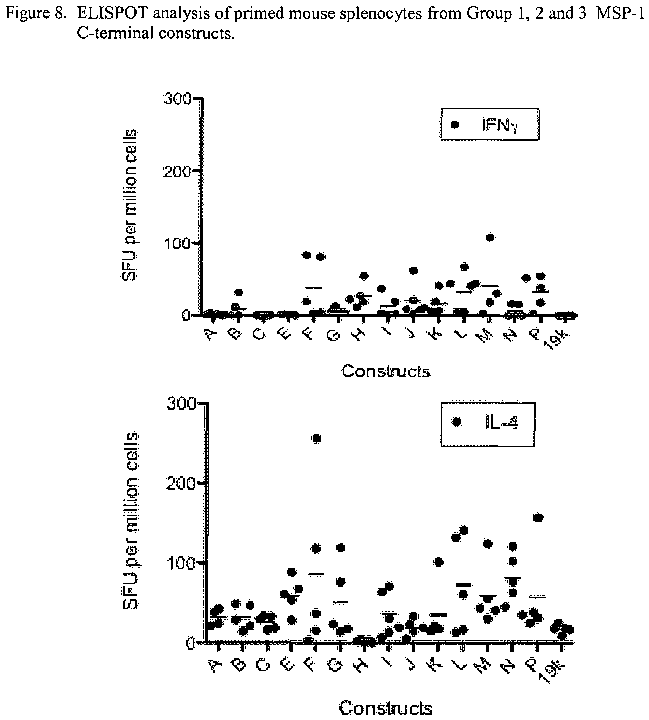

The immunogenicity of the *Drosophila* expressed MSP-1 C-terminal subunit proteins, both Groups 1 and 2 were on the basis of cytokine production from primed mouse splenocytes using the ELISPOT method. The immunization of mice was conducted as described in Example 3. Following the immunization course, a portion of mice from each group were sacrificed 7 days after the final dose and their spleens were removed. Splenocytes were cultured and used for cytokine analysis using the ELISPOT method. The cultured splenocytes for each immunized group was stimulated with the same purified preparation of antigen that was used for the immunization. Following antigen stimulation the cells were analyzed for the production of interferon-gama and interlukin-4. The results of this analysis are presented in Figure 8.

Based on the ELISPOT analysis of Group 1 and Group 2 MSP-1 C-terminal subunit proteins is can be seen that there is a clear distinction between the two groups on the basis of interferon-gama and IL-4 production. Splenocytes primed with Group 1 constructs result in strong IL-4 responses when stimulated and generally result in weak interferon-gama responses. On the other hand, splenocytes primed with Group 2 constructs result in an opposite response when stimulated, strong interferon-gama responses and weak IL-4 responses. These results establish a marked difference between the two groups of constructs, but the nature of this difference in not known at this time. Although the mechanism is not established, there is a strong possibility that the removal of the p33-7 epitope in the Group 2 constructs plays a role in the shift in strong IL-4 response in Group 1 to Group a weak IL-4 response in Group 2. Furthermore, the data supports the fact that multiple levels of experimentation are needed to understand the immune response required to elicit appropriate responses.

### EXAMPLE 6

### Evaluation of the Immunogenicity of MSP-1 C-terminal Subunit Proteins in Rabbits

As the ultimate goal is to determine which MSP-1 C-terminal constructs result in strong and consistent parasite growth inhibition antibodies, selected constructs were used to immunize rabbits in order to conduct this analysis. Constructs were selected based on the results presented in Examples 3, 5 and 6.

Groups of New Zealand White rabbits were immunized as described in Example 3. Parasite growth inhibition assays were conducted as described in Example 3.

The results are presented in the Table X below.

**Table I. In vitro parasite growth inhibition data and antibody titers from rabbits immunized with MSP1 C-terminal constructs**

| Rabbit Sera (Quaternary Bleeds) | | % Parasite growth inhibition^{@} | Reciprocal ELISA Antibody Titer | | | |
|---|---|---|---|---|---|---|
| | | | MSP1-42 | | MSP1-19 | |
| | | | Individual | Mean* | Individual | Mean* |
| Construct-A | Rbt #1 | 75% | 624,000 | | 93,000 | |
| | Rbt #2 | 37% | 361,000 | 482,000 | 23,000 | 43,000 |
| | Rbt #3 | 24% | 498,000 | | 36,000 | |
| Construct-B | Rbt #1 | 66% | 27,000 | | 161,000 | |
| | Rbt #2 | 26% | 156,000 | 101,000 | 137,000 | 139,000 |
| | Rbt #3 | 0% | 245,000 | | 121,000 | |
| Construct-C | Rbt #1 | 10% | 2,490,000 | | 254,000 | |
| | Rbt #2 | 32% | 948,000 | 1,440,000 | 385,000 | 365,000 |
| | Rbt #3 | 22% | 1,265,000 | | 498,000 | |
| Construct-F | Rbt #1 | 0% | 124,000 | | 833,000 | |
| | Rbt #2 | 0% | 146,000 | 203,719 | 328,000 | 554,499 |
| | Rbt #3 | 0% | 467,000 | | 624,000 | |
| Construct-G | Rbt #1 | 58% | 93,000 | | 43,000 | |
| | Rbt #2 | 58% | 125,000 | 218,000 | 133,000 | 146,000 |
| | Rbt #3 | 94% | 889,000 | | 548,000 | |
| Construct-H | Rbt #1 | 31% | 79,000 | | 27,000 | |
| | Rbt #2 | 71% | 113,000 | 340,000 | 28,000 | 111,000 |
| | Rbt #3 | 53% | 5,218,000 | | 1,804,000 | |
| Construct -I | Rbt #1 | 56% | 137,000 | | 117,000 | |
| | Rbt #2 | 0% | 54,000 | 55,000 | 93,000 | 74,000 |
| | Rbt #3 | 0% | 22,000 | | 37,000 | |
| | Rbt #1 | 0% | 156,000 | 180,000 | 202,000 | 220,000 |
| Construct-K | Rbt #2 | 0% | 253,000 | | 172,000 | |
| | Rbt #3 | 0% | 147,000 | | 307,000 | |
| | Rbt #1 | 26% | 110,000 | | 223,000 | |
| Construct-L | Rbt #2 | 56% | 190,000 | 169,000 | 190,000 | 214,000 |
| | Rbt #3 | 0% | 230,000 | | 230,000 | |
| | Rbt #1 | 85% | 125,000 | | 109,000 | |
| Construct -N | Rbt #2 | 78% | 146,000 | 133,000 | 121,000 | 109,000 |
| | Rbt #3 | 66% | 129,000 | | 99,000 | |
| | Rbt #1 | 17% | 88,000 | | 59,000 | |
| Construct-O | Rbt #2 | 6% | 497,000 | 148,582 | 621,000 | 146,034 |
| | Rbt #3 | 11% | 75,000 | | 85,000 | |
| | Rbt #1 | 60% | 1,252,000 | | 140,000 | |
| MSP1-42 | Rbt #2 | 0% | 1,024,000 | 1,198,000 | 317,000 | 179,000 |
| | Rbt #3 | 56% | 1,338,000 | | 129,000 | |

| | | | | | | |
|---|---|---|---|---|---|---|
| ^{@}Mean of two growth inhibition assays ^{*} Geometric mean of antibody titers | | | | | | |

### Other Publications

Al-Yaman, et al. (1996) Am. J. Trop. Med. Hyg. 54:443-448.
Aucouturier et al. (2002) Expert Rev Vaccines 1(1):111-8.
Bernard et al. (1994) Cytotechnology 15:139-144.
Bin et al. (1996) Biochem. J. 313:57-64.
Blackman et al. (1990) J. Exp. Medicine 172:379-382.
Blackman et al. (1991) Mol. Biochem. Parasitology 49:29-34.
Blackman et al. (1992) Mol. Biochem. Parasitology 50:307-316.
Burghaus et al. (1996) Infect Immun. 64(9):3614-9.
Bums et al. (1988) PNAS 85(2):602-6.
Calvo-Calle et al. (1997) J Immunol. 159(3):1362-73.
Cavanagh et al. (2001) Infect Immun. 69(2):1207 11.
Chappel et al. (1993) Mol. Biochem. Parasitology. 60:303-312.
Chang et al. (1992) J. Immuno. 149:548-555.
Chang et al. (1996) Infect. Immun. 64:253-261.
Cheung et al. (1986) PNAS 83:8328-8332.
Culp et al. (1991) Biotechnology 9:173-177.
Daly et al. (1996) Infect Immun. 64:2602-8.
Darko et al. (2005) Infect Immun. 73(1):287-97.
Demotz et al. (1989) J Immunol. 142(2):394-402.
De Souza et al. (1996) Infect Immun. 64(9):3532-6.
Egan et al. (1996) J.Infect.Dis. 173:765-769.
Etlinger et al. (1991) Infect. Immun. 59:3498-3503.
Good et al. (1998) Ann. Rev. Immunonol. 16:57-87.
Guevara et al. (1997) JExp.Med. 186:1689-1699.
Hall et al. (1984) Mol. Biol. Parasitology 11:61-81.
Herrera et al. (1990) PNAS 87:4017-4021.
Hirunpetcharat et al. (1997) J Immunol. 159(7):3400-11.
Holder et al. (1987) Parasitology 94:199-208.
Holder et al. (1988) Parasite Immunology 10:607-617.
Hui et al. (1987) Exp.Parasitol 64:519-522.
Hui (1994) Am J Trop Med Hyg. 50(4 Suppl):41-51.
Huaman et al. (2008) J Immunol 180:1451-1461 (2008).
Incardona et al. (1996) Mol. Biol. of the Cell 7:595-611.
Ivey-Hoyle et al. (1991) PNAS Jan 15;88(2):512-6.
Kaslow et al. (1994) Mol.Biochem.Parasitol. 63:283-289.
Kumar et al. (1995) Molecular Medicine 1:325-332.
Kumar et al. (2000) Infect Immun. 68:2215-2223. ,
Lee et al. (2001) Am J Trop Med Hyg 64:194-203.
Ling et al. (1997) Vaccine 15(14):1562-7.
Lyon et al. (1986) PNAS 83:2989-2993.
Malhotra et al. (2008) J Immunol. 180:3383-3390.
Margalit et al. (1987) J Immunol. Apr 1;138(7):2213-29.
Mcbride et al. (1987) Mol. Biochem. Parasitology. 23:71-84.
Melquist et al. (1998) Biochemistry. 37:6833-6837.
Menendez-Arias et al. (1990) Comput Appl Biosci. Apr;6(2):101-5.
Modis et al. (2003) PNAS 100(12):6986-91.
Morgan et al. (2004) Mol. Biochem. Parasitol. 138:29-36.
Nwuba et al. (2002) Infect.Immun. 70:5328-5331.
Ogutu et al. (2009) PLoS One. 4(3):e4708.
Patarroyo et al. (1987) Nature 328:629-632.
Perera et al. (1998) Infect Immun. 66(4):1500-6.
Pirson et al. (1985) J. Immunology 134:1946-1951.
Riley et al. (1992) Parasite Immunol. 14:321-337.
Schneider (1972) Exp. Morphol. 27:353-356.
Shai et al. (1995) Parasite Immunology 17:269-275.
Shi et al. (1996) Infect. Immun. 64:2716-2723.
Siddiqui et al. (1986) Infect. Immun. 52(1):314-318.
Siddiqui et al. (1987) PNAS 84:3014-3018.
Singh et al. (2001) Bioinformatics Dec;17(12):1236-7.
Stowers et al. (2001) Trends Parasitol. 17(9):415-9.
Stowers et al. (2001) Infect Immun. 69(3):1536-46.
Sturniolo et al. (1999) Nat Biotechnol. 1999 Jun;17(6):555-61.
Tian et al. (1996) J Immunol 157:1176-1183.
Udhayakumar et al. (1995) J Immunol. 154(11):6022 30.
Uthaipibull et al. (2001) J.Mol.Biol. 307:1381-1394.
Van der Straten et al. (1989) Methods in Mol. and Cell. Biol. 1:1-8.
WHO. (2008) "World malaria report 2008*".*
Yang et al. (1999) Infect Immun. 67(1):342-9.

### SEQUENCE LISTING

**SEQ. ID. No: 1** Construct A 
         10        20        30        40        50        60
---------+---------+---------+---------+---------+---------+
AHVKITKLSDLKAIDDKIDLFKNHNDFDAIKKLINDDTKKDMLGKLLSTGLVQNFPNTII
         70        80        90       100       110       120
---------+---------+---------+---------+---------+---------+
SKLIEGKFQDML
**SEQ. ID. No: 2** Construct B 
        10        20        30        40        50        60
---------+---------+---------+---------+---------+---------+
LKPLAGVYRSLKKQGGGGTGAHVKITKLSDLKAIDDKIDLFKNHNDFDAIKKLINDDTKK
        70        80        90       100       110       120
---------+---------+---------+---------+---------+---------+
DMLGKLLSTGLVQNFPNTIISKLIEGKFQDML
**SEQ. ID. No: 3** Construct C 
         10        20        30        40        50        60
---------+---------+---------+---------+---------+---------+
MLGKLLSTGLVQNFPNTIISKLIEGKFQDML
**SEQ. ID. No: 4** Construct D 
         10        20        30        40        50        60
---------+---------+---------+---------+---------+---------+
KFLPFLTNIETLYNNLVNKIDDYLINLKAKINDCNVEKDEAHVKITKLSDLKAIDDKIDL
         70        80        90       100       110       120
---------+---------+---------+---------+---------+---------+
FKNHNDFDAIKKLINDDTKKDMLGKLLSTGLVQNFPNTIISKLIEGKFQDML
**SEQ. ID. No: 5** Construct E 
         10        20        30        40        50        60
---------+---------+---------+---------+---------+---------+
IKKVIKEEKEKFPSSPPTTPPSPAKTDEQKKESKFLPFLTNIETLYNNLVNKIDDYLINL
         70        80        90       100       110       120
---------+---------+---------+---------+---------+---------+
KAKINDCNVEKDEAHVKITKLSDLKAIDDKIDLFKNHNDFDAIKKLINDDTKKDMLGKLL
        130       140       150       160       170       180
---------+---------+---------+---------+---------+---------+
STGLVQNFPNTIISKLIEGKFQDML
**SEQ. ID. No: 6** Construct F 
10        20        30        40        50        60
---------+---------+---------+---------+---------+---------+
KFAQEGISYYEKVLAKYKDDLESIKKVIKEEKEKFPSSPPTTPPSPAKTDEQKKESKFLP
        70        80        90       100       110       120
---------+---------+---------+---------+---------+---------+
FLTNIETLYNNLVNKIDDYLINLKAKINDCNVEKDEAHVKITKLSDLKAIDDKIDLFKNH
       130       140       150       160       170       180
---------+---------+---------+---------+---------+---------+
NDFDAIKKLINDDTKKDMLGKLLSTGLVQNFPNTIISKLIEGKFQDML
**SEQ. ID. No: 7** Construct G 
         10        20        30        40        50        60
---------+---------+---------+---------+---------+---------+
NTLLKSYKYIKESVENDIKFAQEGISYYEKVLAKYKDDLESIKKVIKEEKEKFPSSPPTT
         70        80        90       100       110       120
---------+---------+---------+---------+---------+---------+
PPSPAKTDEQKKESKFLPFLTNIETLYNNLVNKIDDYLINLKAKINDCNVEKDEAHVKIT
        130       140       150       160       170       180
---------+---------+---------+---------+---------+---------+
KLSDLKAIDDKIDLFKNHNDFDAIKKLINDDTKKDMLGKLLSTGLVQNFPNTIISKLIEG
        190       200
---------+---------+---------+---------+---------+---------+
KFQDML
**SEQ. ID. No: 8** Construct H 
         10        20        30        40        50        60
---------+---------+---------+---------+---------+---------+
AISVTMDNILSGFENEYDVIYLKPLAGVYRSLKKQIEKNIFTFNLNLNDILNSRLKKRKY
         70        80        90       100       110       120
---------+---------+---------+---------+---------+---------+
FLDVLESDLMQFKHISSNEYIIEDSFKLLNSEQKNTLLKSYKYIKESVENDIKFAQEGIS
        130       140       150       160       170       180
---------+---------+---------+---------+---------+---------+
YYEKVLAKYKDDLESIKKVIKEEKEKFPSSPPTTPPSPAKTDEQKKESKFLPFLTNIETL
        190       200
---------+---------+---------+---------+---------+---------+
LINDDTKKDMLGGGGGTG
**SEQ. ID. No: 9** Construct I 
         10        20        30        40        50        60
---------+---------+---------+---------+---------+---------+
AISVTMDNILSGFENEYDVIGGGGSGVEKDEAHVKITKLSDLKAIDDKIDLFKNHNDFDA
         70        80        90       100       110       120
---------+---------+---------+---------+---------+---------+
IKKLINDDTKKDMLGGGGGTG
**SEQ. ID. No: 10** Construct J 
         10        20        30        40        50        60
---------+---------+---------+---------+---------+---------+
AISVTMDNILSGFENEYDVIYLKPLAGVYRSLKKQIEKNIGGGGSGVEKDEAHVKITKLS
         70        80        90       100       110       120
---------+---------+---------+---------+---------+---------+
DLKAIDDKIDLFKNHNDFDAIKKLINDDTKKDMLGGGGTG
**SEQ. ID. No: 11** Construct K 
         10        20        30        40        50        60
---------+---------+---------+---------+---------+---------+
AQEGISYYEKVLAKYKDDLESIKKVIKEEKEKFPSSGGGGSGVEKDEAHVKITKLSDLKA
         70        80        90       100       110       120
---------+---------+---------+---------+---------+---------+
IDDKIDLFKNHNDFDAIKKLINDDTKKDMLGGGGGTG
**SEQ. ID. No: 12** Construct L 
         10        20        30        40        50        60
---------+---------+---------+---------+---------+---------+
ISSNEYIIEDSFKLLNSEQKNTLGGGGSGVEKDEAHVKITKLSDLKAIDDKIDLFKNHND
         70        80        90       100       110       120
---------+---------+---------+---------+---------+---------+
FDAIKKLINDDTKKDMLGGGGGTG
**SEQ. ID. No: 13** Construct M 
         10        20        30        40        50        60
---------+---------+---------+---------+---------+---------+
NLVNKIDDYLINLKAKINDCNVEKDEAHVKITKLSDLKAIDDKIDLFKNHNDFDAIKKLI
         70        80        90       100       110       120
---------+---------+---------+---------+---------+---------+
NDDTKKDMLGGGGGTG
**SEQ. ID. No: 14** Construct N 
         10        20        30        40        50        60
---------+---------+---------+---------+---------+---------+
DNILSYLKPLAGVYRSLKKQILNSRLVLESDLKYKDDLESIKKVKFLPFLTNIETL
**SEQ. ID. No: 15** Construct O 
        10        20        30        40        50        60
---------+---------+---------+---------+---------+---------+
AISVTMGFENEYDVIEKNIFTFNLNLNDIKKRKYFLDMQFKHISSNEYIIEDSFKLLNSE
        70        80        90       100       110       120
---------+---------+---------+---------+---------+---------+
QKNTLLKSYKYIKESVENDIKFAQEGISYYEKVLAVIKEEKEKFPSSPPTTPPSPAKTDE
       130       140       150       160       170       180
---------+---------+---------+---------+---------+---------+
QKKESYNNLVNKIDDYLINLKAKINDCNVEKDEAHVKITKLSDLKAIDDKIDLFKNHNDF
       190       200       210       220       230       240
---------+---------+---------+---------+---------+---------+
DAIKKLINDDTKKDMLGKLLSTGLVQNFPNTIISKLIEGKFQDML
**SEQ. ID. No: 16** Construct P 
         10        20        30        40        50        60
---------+---------+---------+---------+---------+---------+
VEKDEAHVKITKLSDLKAIDDKIDLFKNHNDFDAIKKLINDDTKKDMLG
**SEQ. ID. No: 17** Construct Q 
10        20        30        40        50        60
---------+---------+---------+---------+---------+---------+
NLVNKIDDYLINLKAKINDCNVEKDEAHVKITKLSDLKAIDDKIDLFKNHNDFDAIKKLI
        70        80        90       100       110       120
---------+---------+---------+---------+---------+---------+
NDDTKKDMLGKLLSTGLVQNFPNTIISKLIEGKFQDML
**SEQ. ID. No: 18** Construct R

10        20        30        40        50        60
---------+---------+---------+---------+---------+---------+
AISVTMDNILSGFENEYDVIGGGGSGISSNEYIIEDSFKLLNSEQKNTLAQEGISYYEKV
        70        80        90       100       110       120
---------+---------+---------+---------+---------+---------+
LAKYKDDLESIKKVIKEEKEKFPSS

## Claims

1. MSP-1 C-terminal recombinant subunit protein with enhanced immunogenic properties comprising:
the p19 core region of the *Plasmodium falciparum* MSP-1 protein of the C-terminal region to which a segment of the p33 region is operably linked, wherein said segment of the p33 region does not include the amino acid sequence LVQNFPNTIISKLIEGK, wherein all animals immunized with said protein elicit an immunologic response that results in parasite growth inhibition activity greater than 50%, and wherein the linked segment of the p33 region is SEQ. ID. NO: 8 or SEQ. ID. NO: 14.

2. The recombinant subunit protein of claim 1, wherein the protein is expressed in a eukaryotic cell culture system.

3. The recombinant subunit protein of claim 2, wherein the cell culture expression system utilizes insect cells.

4. The recombinant subunit protein of claim 3, wherein the insect cells are *Drosophila* S2 cells.

5. Vaccine formulations comprising MSP-1 C-terminal subunit proteins of any of claims 1-4.

6. Vaccine formulations comprising MSP-1 C-terminal subunit proteins of any of claims 1-4 for use in eliciting protection from malaria infection.

7. The vaccine formulations of claims 5 or 6, wherein the segment of the p33 region segment of the MSP-1 C-terminal subunit protein is SEQ. ID. NO: 8 or SEQ. ID. NO: 14.

## Patentansprüche

1. C-terminale rekombinante MSP-1-Proteinuntereinheit mit verstärkten immunogenen Eigenschaften, umfassend:
die p19-Kernregion des MSP1-Proteins von *Plasmodium falciparum* der C-terminalen Region, mit der ein Abschnitt der p33-Region funktionell verknüpft ist, wobei der Abschnitt der p33-Region die Aminosäuresequenz LVQNFPNTIISKLIEGK nicht umfasst, wobei alle Tiere, die mit dem Protein immunisiert sind, eine immunologische Reaktion hervorrufen, die zu einer hemmenden Aktivität auf das Parasitenwachstum von mehr als 50% führt und wobei der verknüpfte Abschnitt der p33-Region SEQ ID NO:8 oder SEQ ID NO:14 ist.

2. Rekombinante Proteinuntereinheit nach Anspruch 1, wobei das Protein in einem Kultursystem von eukaryotischen Zellen exprimiert wird.

3. Rekombinante Proteinuntereinheit nach Anspruch 2, wobei das Zellkultur-Expressionssystem Insektenzellen verwendet.

4. Rekombinante Proteinuntereinheit nach Anspruch 3, wobei die Insektenzellen *Drosophila* S2-Zellen sind.

5. Impfstoffformulierungen, die C-terminale MSP-1-Proteinuntereinheiten nach einem der Ansprüche 1 bis 4 umfassen.

6. Impfstoffformulierungen, die C-terminale MSP-1-Proteinuntereinheiten nach einem der Ansprüche 1 bis 4 umfassen, zur Verwendung beim Hervorrufen eines Schutzes gegen Malariainfektion.

7. Impfstoffformulierungen nach Anspruch 5 oder 6, wobei der Abschnitt des p33-Regionabschnitts der C-terminalen MSP-1-Proteinuntereinheit SEQ ID NO:8 oder SEQ ID NO:14 ist.

## Revendications

1. Protéine de sous-unité recombinante C-terminale de MSP-1 possédant des propriétés immunogènes améliorées comprenant :
la région de noyau p19 de la protéine MSP-1 de *Plasmodium falciparum* de la région C-terminale en liaison fonctionnelle avec un segment de la région p33, où ledit segment de la région p33 ne comprend pas la séquence d'acides aminés LVQNFPNTIISKLIEGK, où tous les animaux immunisés avec ladite protéine génèrent une réponse immunologique qui entraîne une activité d'inhibition de la croissance de parasites supérieure à 50 %, et où le segment lié de la région p33 est SEQ ID NO: 8 ou SEQ ID NO: 14.

2. Protéine de sous-unité recombinante selon la revendication 1, où la protéine est exprimée dans un système de culture cellulaire eucaryote.

3. Protéine de sous-unité recombinante selon la revendication 2, où le système d'expression de culture cellulaire utilise des cellules d'insecte.

4. Protéine de sous-unité recombinante selon la revendication 3, où les cellules d'insecte sont des cellules S2 de *Drosophila.*

5. Formulations de vaccin comprenant des protéines de sous-unité C-terminale de MSP-1 selon l'une quelconque des revendications 1 à 4.

6. Formulations de vaccin comprenant des protéines de sous-unité C-terminale de MSP-1 selon l'une quelconque des revendications 1 à 4 destinées à être utilisées pour obtenir une protection contre une infection par la malaria.

7. Formulations de vaccin selon les revendications 5 ou 6, dans lesquelles le segment du segment de la région p33 de la protéine de sous-unité C-terminale de MSP-1 est SEQ ID NO: 8 ou SEQ ID NO: 14.
